(19) Europäisches Patentamt
European Patent Office
Office européen des brevets

(11) **EP 3 689 005 B1**

(12) **EUROPÄISCHE PATENTSCHRIFT**

(45) Veröffentlichungstag und Bekanntmachung des Hinweises auf die Patenterteilung:
**17.04.2024 Patentblatt 2024/16**

(21) Anmeldenummer: **18746690.9**

(22) Anmeldetag: **26.07.2018**

(51) Internationale Patentklassifikation (IPC):
**H04R 25/00** (2006.01)   **A61N 1/36** (2006.01)

(52) Gemeinsame Patentklassifikation (CPC):
**H04R 25/356; A61N 1/36038;** A61N 1/0541;
H04R 2225/43; H04S 2420/07

(86) Internationale Anmeldenummer:
**PCT/EP2018/070368**

(87) Internationale Veröffentlichungsnummer:
**WO 2019/057370 (28.03.2019 Gazette 2019/13)**

(54) **VERFAHREN UND VORRICHTUNG ZUR RECHNERGESTÜTZTEN VERARBEITUNG VON AUDIOSIGNALEN**

METHOD AND APPARATUS FOR COMPUTER-ASSISTED PROCESSING OF AUDIOSIGNALS

PROCÉDÉ ET DISPOSITIF DE TRAITEMENT DE SIGNAUX AUDIO ASSISTÉ PAR ORDINATEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.09.2017 DE 102017216972**

(43) Veröffentlichungstag der Anmeldung:
**05.08.2020 Patentblatt 2020/32**

(73) Patentinhaber: **Carl von Ossietzky Universität Oldenburg**
**26129 Oldenburg (DE)**

(72) Erfinder:
• **SCHÄDLER, Marc René**
**26121 Oldenburg (DE)**
• **KOLLMEIER, Birger**
**26129 Oldenburg (DE)**
• **WARZYBOK, Anna**
**28355 Bremen (DE)**
• **HÜLSMEIER-REINEKE, David**
**26131 Oldenburg (DE)**
• **BUHL, Mareike**
**26127 Oldenburg (DE)**

(74) Vertreter: **Fink Numrich Patentanwälte PartmbB**
**Paul-Gerhardt-Allee 24**
**81245 München (DE)**

(56) Entgegenhaltungen:
**EP-A2- 1 912 470     US-A1- 2011 150 229**

• **SCHÄDLER MARC RENÉ ET AL: "A simulation framework for auditory discrimination experiments: Revealing the importance of across-frequency processing in speech perception", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, Bd. 139, Nr. 5, 13. Mai 2016 (2016-05-13), Seiten 2708-2722, XP012207680, ISSN: 0001-4966, DOI: 10.1121/1.4948772 [gefunden am 1901-01-01]**

**Beschreibung**

Die Erfindung betrifft ein Verfahren und eine Vorrichtung zur rechnergestützten Verarbeitung von Audiosignalen.

Aus dem Stand der Technik sind verschiedene Verfahren zur sog. Dynamikkompression von Audiosignalen bekannt. Ein bevorzugter Anwendungsbereich dieser Verfahren ist die Wandlung von Audiosignalen in Hörgeräten. Mittels der Dynamikkompression wird der Wertebereich der Signalpegel eines Audiosignals in einen verkleinerten Wertebereich abgebildet, so dass leise Geräusche besser gehört werden können und laute Geräusche als nicht zu laut empfunden werden. Die Kompression erfolgt in Abhängigkeit von den im Audiosignal enthaltenen Frequenzen.

Bekannte Verfahren zur Dynamikkompression sind beispielsweise in den Dokumenten DE 10 2006 047 694 B4, DE 10 2004 044 565 A1 sowie WO 2015/113601 A1 beschrieben.

Die herkömmlichen Verfahren zur Dynamikkompression weisen den Nachteil auf, dass Sprachanteile im Audiosignal meist genauso verstärkt werden wie Störgeräusche oder dass es zu Verzerrungen im erzeugten Ausgangssignal kommt. Demzufolge wird bei der Verwendung dieser Verfahren in Hörgeräten oftmals keine zufriedenstellende Signalqualität erreicht.

Die Druckschrift EP 1 912 470 A2 offenbart ein Verfahren zur Dynamikkompression eines Audiosignals bei Hörvorrichtungen, bei dem ein Modulationsspektrum des Audiosignals mittels einer Fast-Fourier-Transformation gewonnen wird und dieses Modulationsspektrum entsprechend einer vorbestimmten Funktion modifiziert wird. Schließlich wird ein modifiziertes bzw. komprimiertes Ausgangssignal aus dem modifizierten Modulationsspektrum rückgewonnen.

Das Dokument SCHÄDLER MARC RENÉ ET AL: "A simulation framework for auditory discrimination experiments: Revealing the importance of across-frequency processing in speech perception", THE JOURNAL OF THE ACOUSTICAL SOCIETY OF AMERICA, Bd. 139, Nr. 5, 13. Mai 2016, Seiten 2708-2722, beschreibt die spektro-temporale Filterung eines Audiosignals, um bestimmte Merkmale in dem Audiosignal zu erkennen.

In der Druckschrift US 2011/0150229 A1 ist ein Verfahren zur Bestimmung eines auditiven Musters beschrieben, das mit einem Audiosegment verknüpft ist. Dabei werden Signalintensitäten von Frequenzanteilen für unterschiedliche Detektorpositionen im menschlichen Innenohr bestimmt.

Aufgabe der Erfindung ist es, ein Verfahren zur rechnergestützten Verarbeitung von Audiosignalen zu schaffen, mit dem Ausgangssignale mit verbesserter Qualität erzeugt werden können.

Diese Aufgabe wird durch das Verfahren gemäß Patentanspruch 1 gelöst. Weiterbildungen der Erfindung sind in den abhängigen Ansprüchen definiert.

Das erfindungsgemäße Verfahren dient zur rechnergestützten Verarbeitung von Audiosignalen. Dabei wird in einem Schritt a) als Eingangssignal ein Audiosignal (d.h. ein Schallwellensignal) in der Form eines Zeitsignals aus Amplitudenwerten (d.h. ein Signal mit Amplituden zu aufeinander folgenden Zeitpunkten) digital erfasst, wobei die im Eingangssignal enthaltenen spektralen Zeitsignale in mehreren Frequenzbändern bestimmt werden, wodurch eine Vielzahl von Signalpegeln (zu aufeinander folgenden Zeitpunkten) für jedes spektrale Zeitsignal erhalten wird. Den jeweiligen Frequenzbändern sind dabei Signalfrequenzen in der Form von entsprechenden Mittenfrequenzen zugeordnet. Vorzugsweise werden die Signalpegel in dB SPL (SPL = Sound Pressure Level) angegeben. Die digitale Erfassung des Audiosignals kann beispielsweise mittels eines Mikrofons mit zugeordnetem Analog-Digital-Wandler erfolgen. Die Extraktion von spektralen Zeitsignalen aus dem Eingangssignal ist an sich aus dem Stand der Technik bekannt und kann beispielsweise mittels einer Vielzahl von Bandpassfiltern erreicht werden, wie auch näher in der detaillierten Beschreibung erläutert ist.

In einem Schritt b) des erfindungsgemäßen Verfahrens wird aus den spektralen Zeitsignalen eine spektro-temporale Repräsentation des Eingangssignals ermittelt, wobei diese spektro-temporale Repräsentation für jeweilige Zeitfenster (aus mehreren aufeinander folgenden Zeitpunkten) eine Vielzahl von Signalpegeln enthält, wobei die Signalpegel in einem Zeitfenster jeweils einer anderen Signalfrequenz zugeordnet sind. In einer einfachen Variante des erfindungsgemäßen Verfahrens kann die spektro-temporale Repräsentation durch Mittelung der Signalpegel des Eingangssignals im jeweiligen Zeitfenster bestimmt werden. Vorzugsweise werden jedoch im Rahmen der Ermittlung der spektro-temporalen Repräsentation ein oder mehrere andere Verarbeitungsschritte durchgeführt, wobei bevorzugte Varianten dieser Verarbeitungsschritte weiter unten beschrieben werden.

In einem Schritt c) des erfindungsgemäßen Verfahrens wird die spektro-temporale Repräsentation einer Modulations-Bandpassfilterung umfassend eine spektrale Modulations-Bandpassfilterung unterzogen, wobei durch die spektrale Modulations-Bandpassfilterung aus mehreren nicht überlappenden und vorzugsweise aneinander angrenzenden spektralen Modulationsfrequenzbändern, denen jeweils ein Frequenzwert in der Form der minimalen spektralen Modulationsfrequenz im entsprechenden Modulationsfrequenzband zugeordnet ist, jeweils die spektrale Variation der im jeweiligen Modulationsfrequenzband enthaltenen Signalanteile entlang der Signalfrequenzen extrahiert wird. Sofern nichts anderes angegeben, wird hier und im Folgenden unter einem Modulationsfrequenzband ein spektrales Modulationsfrequenzband verstanden. Spektral bedeutet dabei, dass die Änderung der Signalanteile in Richtung der Signalfrequenzen betrachtet wird. Die spektrale Modulations-Bandpassfilterung, welche ein wesentliches Merkmal der Erfindung darstellt, berücksichtigt die Wechselwirkung zwischen den unterschiedlichen spektralen Zeitsignalen.

**EP 3 689 005 B1**

Als Ergebnis der spektralen Modulations-Bandpassfilterung erhält man für jedes Modulationsfrequenzband eine oder mehrere erste Modulationssignal-Repräsentationen, wobei jede erste Modulationssignal-Repräsentation für jeweilige Zeitfenster eine Vielzahl von Modulationssignalpegeln enthält, wobei die Modulationssignalpegel in einem Zeitfenster jeweils einer anderen Signalfrequenz zugeordnet sind. Sofern in Schritt c) nur eine spektrale Modulations-Bandpassfilterung durchgeführt wird, erhält man für jedes Modulationsfrequenzband nur eine einzelne erste Modulationssignal-Repräsentation. Im Falle, dass zusätzlich auch eine temporale Modulations-Bandpassfilterung durchgeführt wird, kann ein jeweiliges Modulationsfrequenzband gegebenenfalls mehrere erste Modulationssignal-Repräsentationen enthalten, von denen zumindest ein Teil temporal bandpass-gefiltert sind.

In einem Schritt d) des erfindungsgemäßen Verfahrens werden die ersten Modulationssignal-Repräsentationen in zweite Modulationssignal-Repräsentationen gewandelt, d.h. aus jeder ersten Modulationssignal-Repräsentation wird eine zweite Modulationssignal-Repräsentation mit gleichem Modulationsfrequenzband wie die erste Modulationssignal-Repräsentation erzeugt. Dabei ist zumindest ein Teil der zweiten Modulationssignal-Repräsentationen gegenüber der entsprechenden ersten Modulationssignal-Repräsentation modifiziert.

Erfindungsgemäß wird in Schritt d) für das Modulationsfrequenzband mit dem niedrigsten Frequenzwert die erste Modulationssignal-Repräsentation mittels einer Kompressions-Funktion in die zweite Modulationssignal-Repräsentation derart gewandelt, dass für jede Signalfrequenz in einem jeweiligen Zeitfenster ein vorgegebener Wertebereich der ersten Modulationssignalpegel auf einen verkleinerten Wertebereich der zweiten Modulationssignalpegel abgebildet wird. Je nach Anwendung kann der verkleinerte Wertebereich innerhalb des vorgegebenen Wertebereichs der ersten Modulationssignalpegel liegen oder mit diesem Wertebereich überlappen. In Spezialfällen kann der verkleinerte Wertebereich auch außerhalb des vorgegebenen Wertebereichs liegen. Der vorgegebene Wertebereich braucht dabei nicht alle Signalpegel umfassen, die in der ersten Modulationssignal-Repräsentation auftreten. Die Kompressions-Funktion wird vorzugsweise ausschließlich auf das Modulationsfrequenzband mit dem niedrigsten Frequenzwert angewendet. Mit dieser Variante wird eine geeignete Kompression in einen gewünschten Zieldynamikbereich gewährleistet.

Erfindungsgemäß wird in Schritt d) ferner zumindest eine zweite Modulationssignal-Repräsentation durch die Multiplikation der entsprechenden ersten Modulationssignal-Repräsentation mit einem Faktor größer Null erhalten. Vorzugsweise werden alle zweiten Modulationssignal-Repräsentationen, außer der Modulationssignal-Repräsentation für das Modulationsfrequenzband mit dem niedrigsten Frequenzwert, über eine solche Modifikation erhalten. In einer weiteren bevorzugten Variante ist der Faktor auf jeden Fall für zumindest eine Modulationssignal-Repräsentation des Modulationsfrequenzbands mit dem zweithöchsten Frequenzwert ≥ 1.

In Schritt e) des erfindungsgemäßen Verfahrens wird eine modifizierte spektro-temporale Repräsentation ermittelt, indem die zweiten Modulationssignal-Repräsentationen derart kombiniert werden, dass für ein jeweiliges Zeitfenster jeder Signalpegel, der in der unmodifizierten spektro-temporalen Repräsentation oberhalb einer vorgegebenen Hörschwelle liegt, in der modifizierten spektro-temporalen Repräsentation auf einen Signalpegel innerhalb eines Zielpegelbereichs abgebildet wird, wobei der Zielpegelbereich von der Signalfrequenz abhängt und durch eine untere Zielhörschwelle nach unten begrenzt ist. Der Begriff der Zielhörschwelle und auch der Hörschwelle ist derart zu verstehen, dass es sich um eine Schwelle mit zugeordneten Signalpegeln handelt, die von einem Normalhörenden gehört werden können. Ferner bezieht sich die weiter unten erwähnte Ruhehörschwelle eines Normalhörenden auf die minimalen Signalpegel, die ein Normalhörender gerade noch hören kann (z.B. nach Norm ISO 226: 2006-04). Analog ist die Ruhehörschwelle eines Schwerhörenden definiert. In einer bevorzugten Variante ist die untere Zielhörschwelle die Ruhehörschwelle eines Schwerhörenden. In einer weiteren bevorzugten Variante ist die vorgegebene Hörschwelle die Ruhehörschwelle eines Normalhörenden oder eine darüber liegende Hörschwelle.

In einem Schritt f) des erfindungsgemäßen Verfahrens wird aus der modifizierten spektro-temporalen Repräsentation ein Ausgangssignal erzeugt, das gegenüber dem Eingangssignal modifiziert ist. Bei Verwendung des Verfahrens in einem Cochlea-Implantat kann die modifizierte spektro-temporale Repräsentation beispielsweise direkt als Signal für eine an sich bekannte Signalkodierung der Elektroden des Implantats verwendet werden. In diesem Fall stellt das kodierte Signal das Ausgangssignal des Schritts f) dar. In einer weiteren bevorzugten Ausführungsform wird in Schritt f) die Differenz zwischen der modifizierten spektro-temporalen Repräsentation und der unmodifizierten spektro-temporalen Repräsentation ermittelt. Diese Differenz wird auf die spektralen Zeitsignale des Eingangssignals in den mehreren Frequenzbändern angewendet, wodurch das modifizierte Ausgangssignal erzeugt wird. Dieses Ausgangssignal kann dann in ein entsprechendes Audiosignal gewandelt werden, z.B. bei Verwendung des erfindungsgemäßen Verfahrens in einem Hörgerät. Im Falle, dass die Differenz in dB angegeben ist, wird unter der Anwendung der Differenz insbesondere ein Verstärken bzw. Abschwächen der Amplitude des Eingangssignals um den Faktor $10^{Differenz/20}$ verstanden.

Das erfindungsgemäße Verfahren zeichnet sich dadurch aus, dass eine Aufteilung der spektralen Zeitsignale eines Audiosignals in spektrale Modulationsfrequenzbänder erfolgt. Dabei macht man sich die Erkenntnis zunutze, dass durch eine geeignete Modifikation dieser spektralen Strukturen eine Verbesserung der Signalqualität vor allem im Hinblick auf die Sprachverständlichkeit erreicht werden kann.

Der oben definierte Zielpegelbereich kann gegebenenfalls nur nach unten durch die untere Zielhörschwelle begrenzt sein. Vorzugsweise ist der Zielpegelbereich jedoch auch durch eine obere Zielhörschwelle nach oben begrenzt.

3

Vorzugsweise entspricht die obere Zielhörschwelle einer Unbehaglichkeitsschwelle eines Schwerhörenden, d.h. einer Schwelle, bei der die Signalpegel derart hoch sind, dass sie von einem Schwerhörenden als unangenehm empfunden werden. Analog gibt es auch eine Unbehaglichkeitsschwelle eines Normalhörenden, d.h. eine Schwelle, bei der die Signalpegel derart hoch sind, dass sie von einem Normalhörenden als unangenehm empfunden werden

In einer bevorzugten Variante des erfindungsgemäßen Verfahrens wird die Wandlung der ersten Modulationssignal-Repräsentationen in zweite Modulationssignal-Repräsentationen in Schritt d) derart durchgeführt, dass alle zweiten Modulationssignal-Repräsentationen, deren jeweiliges Modulationsfrequenzband Modulationsfrequenzen von größer gleich 0,25/ERB enthält, gegenüber den entsprechenden ersten Modulationssignal-Repräsentationen unmodifiziert bleiben oder verstärkt werden. Dabei kann gegebenenfalls ein Teil dieser Bänder unmodifiziert bleiben, wohingegen ein anderer Teil der Bänder verstärkt wird. Bei dieser Variante macht man sich die Erkenntnis zunutze, dass für die Spracherkennung die schmalen spektralen Strukturen besonders wichtig sind, so dass diese erhalten bleiben sollen. Es wird somit eine bessere Sprachverständlichkeit erreicht.

In einer besonders bevorzugten Ausführungsform des erfindungsgemäßen Verfahrens wird die Wandlung der ersten Modulationssignal-Repräsentationen in zweite Modulationssignal-Repräsentationen in Schritt d) derart durchgeführt, dass für das Modulationsfrequenzband mit dem höchsten Frequenzwert jede zweite Modulationssignal-Repräsentation mit der ersten Modulationssignal-Repräsentation identisch ist und eine oder mehrere zweite Modulationssignal-Repräsentationen umfassend zumindest eine zweite Modulationssignal-Repräsentation für das Modulationsfrequenzband mit dem zweithöchsten Frequenzwert und/oder jede zweite Modulationssignal-Repräsentation für das Modulationsfrequenzband mit dem niedrigsten Frequenzwert gegenüber der entsprechenden ersten Modulationssignal-Repräsentation modifiziert ist, wobei die zumindest eine zweite Modulationssignal-Repräsentation für das Modulationsfrequenzband mit dem zweithöchsten Frequenzwert vorzugsweise verstärkt ist. Hierdurch wird ebenfalls eine Verbesserung der Sprachverständlichkeit für das modifizierte Ausgangssignal erreicht.

In einer weiteren Variante des erfindungsgemäßen Verfahrens werden die zweiten Modulationssignal-Repräsentationen in Schritt e) derart kombiniert, dass zu der oder den zweiten Modulationssignal-Repräsentationen für das Modulationsfrequenzband mit dem niedrigsten Frequenzwert zumindest eine zweite Modulationssignal-Repräsentation für das Modulationsfrequenzband mit dem zweithöchsten Frequenzwert und alle zweiten Modulationssignal-Repräsentationen für das Modulationsfrequenzband mit dem höchsten Frequenzwert hinzuaddiert werden und anschließend schrittweise die restlichen zweiten Modulationssignal-Repräsentationen in der Reihenfolge hin zu Modulationsfrequenzbändern mit niedrigen Frequenzwerten hinzuaddiert werden, wobei vor dem Hinzuaddieren einer vorgegebenen Anzahl von Modulationssignalpegeln einer zweiten Modulationssignal-Repräsentation aus den restlichen zweiten Modulationssignal-Repräsentationen für ein jeweiliges Zeitfenster überprüft wird, ob eine Zulässigkeitsbedingung für die vorgegebene Anzahl von Modulationssignalpegeln verletzt ist. Je nach Ausgestaltung kann die vorgegebene Anzahl einen Teil oder ggf. auch alle Modulationssignalpegel der jeweiligen zweiten Modulationssignal-Repräsentation betreffen. Zum Beispiel kann die Zulässigkeitsbedingung für einen Teil der Signalfrequenzen erfüllt sein, wohingegen sie für einen anderen Teil der Signalfrequenzen nicht erfüllt ist. Das Hinzuaddieren der vorgegebenen Anzahl für das jeweilige Zeitfenster (für alle noch hinzuaddierbaren zweiten Modulationssignal-Repräsentationen) wird bei Verletzen der Zulässigkeitsbedingung beendet, wobei die Zulässigkeitsbedingung dann verletzt ist, wenn das Hinzuaddieren des Modulationssignalpegels in einem jeweiligen Zeitfenster für zumindest eine Signalfrequenz in der vorgegebenen Anzahl zu einem Signalpegel außerhalb des oben definierten Zielpegelbereichs führt, welcher vor dem Hinzufügen noch innerhalb des Zielpegelbereichs lag. Mit dieser Variante kann auf einfache Weise sichergestellt werden, dass die Signalpegel im Zielpegelbereich bleiben.

In einer bevorzugten Variante des erfindungsgemäßen Verfahrens ist der vorgegebene Wertebereich nach unten durch eine untere Hörschwelle begrenzt, welche die Ruhehörschwelle eines Normalhörenden oder eine darüber liegende Hörschwelle ist, und der verkleinerte Wertebereich ist nach unten durch eine untere Hörschwelle begrenzt, welche vorzugsweise die Ruhehörschwelle eines Schwerhörenden ist. Dabei sind der vorgegebene Wertebereich und der verkleinerte Wertebereich vorzugsweise auch durch eine obere Hörschwelle nach oben begrenzt, wobei die obere Hörschwelle für den vorgegebenen Wertebereich vorzugsweise eine Unbehaglichkeitsschwelle eines Normalhörenden ist und die obere Hörschwelle für den verkleinerten Wertebereich vorzugsweise eine Unbehaglichkeitsschwelle eines Schwerhörenden ist. Diese Variante des erfindungsgemäßen Verfahrens eignet sich insbesondere zur Verwendung in Hörhilfen, wie Hörgeräten oder Cochlea-Implantaten oder Hirnstamm-Implantaten.

In einer weiteren bevorzugten Variante des erfindungsgemäßen Verfahrens werden in Schritt b) die Signalpegel eines jeweiligen spektralen Zeitsignals derart verändert, dass die Signalpegel innerhalb eines jeweiligen Zeitfensters durch einen Wert zwischen einschließlich dem Maximalwert und einschließlich dem Minimalwert der Signalpegel innerhalb des jeweiligen Zeitfensters, vorzugsweise durch den Maximalwert, ersetzt werden, wodurch einem Zeitfenster ein einzelner Signalpegel eines jeweiligen spektralen Zeitsignals zugeordnet wird. Sofern keine weitere Signalverarbeitung in Schritt b) durchgeführt wird, stellen diese einzelnen Signalpegel die Signalpegel der spektro-temporalen Repräsentation dar. Diese Variante des erfindungsgemäßen Verfahrens ermöglicht eine Rechenzeit- und Datenreduktion.

In einer bevorzugten Variante der soeben beschriebenen Ausführungsform werden in Schritt b) die einzelnen Signalpegel der jeweiligen spektralen Zeitsignale für die jeweiligen Zeitfenster derart angepasst, dass der ursprüngliche Signalpegel eines jeweiligen spektralen Zeitsignals über eine vorbestimmte Anzahl von mehreren Zeitfenstern konstant gehalten wird und hierdurch aktualisierte ursprüngliche Signalpegel erzeugt werden, solange sich der ursprüngliche Signalpegel in der vorbestimmten Anzahl von mehreren Zeitfenstern nicht erhöht, wobei im Falle, dass sich der ursprüngliche Signalpegel für ein Zeitfenster in der vorbestimmten Anzahl von mehreren Zeitfenstern erhöht, der erhöhte Signalpegel für das Zeitfenster als aktualisierter ursprünglicher Signalpegel weiterverwendet wird, und wobei im Falle, dass sich der ursprüngliche Signalpegel in der vorbestimmten Anzahl von mehreren Zeitfenstern nicht erhöht, der ursprüngliche Signalpegel in den Zeitfenstern, die auf die vorbestimmte Anzahl von mehreren Zeitfenstern folgen, sukzessive erniedrigt wird und hierdurch aktualisierte ursprüngliche Signalpegel erzeugt werden, bis der nächste ursprüngliche Signalpegel für ein Zeitfenster höher wird als der erniedrigte Signalpegel, woraufhin der höhere Signalpegel für das Zeitfenster als aktualisierter ursprünglicher Signalpegel weiterverwendet wird. Ohne weitere Signalverarbeitung in Schritt b) stellen die derart erhaltenen Signalpegel die Signalpegel der spektro-temporalen Repräsentation dar. Mit dieser Variante der Erfindung kann eine Verbesserung der Sprachverständlichkeit im modifizierten Ausgangssignal erreicht werden.

In einer weiteren Variante des erfindungsgemäßen Verfahrens, welche mit jeder der beiden zuvor beschriebenen Ausführungsformen kombiniert werden kann, wird in einem jeweiligen Zeitfenster für jeden einzelnen Signalpegel (gegebenenfalls angepasst basierend auf der zuvor beschriebenen Ausführungsform) der spektralen Zeitsignale in einer Kopie aller Signalpegel des jeweiligen Zeitfensters eine vorbestimmte Anzahl von Signalpegeln, welche spektral gemäß der Reihenfolge der Signalfrequenzen zu dem jeweiligen einzelnen Signalpegel benachbart sind, gedämpft. Dabei ist die Dämpfung umso höher, je größer der spektrale Abstand eines Signalpegels aus der vorbestimmten Anzahl von Signalpegeln zu dem jeweiligen einzelnen Signalpegel gemäß der Reihenfolge der Signalfrequenzen ist. Im Falle, dass der Maximalwert der gedämpften Signalpegel der vorbestimmten Anzahl von Signalpegeln größer als der jeweilige einzelne Signalpegel ist, ersetzt der Maximalwert den jeweiligen einzelnen Signalpegel in den ursprünglichen Signalpegeln (d.h. nicht in der Kopie der Signalpegel). Ist dies nicht der Fall, bleibt der jeweilige einzelne Signalpegel unverändert. Mit dieser Variante kann die Qualität des Ausgangssignals weiter verbessert werden. Insbesondere werden Töne, die von einem Normalhörenden nicht gehört werden, weniger verstärkt.

Die im erfindungsgemäßen Verfahren durchgeführte Modulations-Bandpassfilterung in Schritt c) kann neben der spektralen Modulations-Bandpassfilterung auch eine temporale Modulations-Bandpassfilterung umfassen. Diese temporale Modulations-Bandpassfilterung wird auf die spektro-temporale Repräsentation angewendet, wobei durch die temporale Modulations-Bandpassfilterung die temporale Variation der in einem oder mehreren Zeitmodulationsfrequenzbändern enthaltenen Signalanteile entlang der aufeinander folgenden Zeitfenster extrahiert wird, so dass eines oder mehrere der ersten Modulationssignale auch temporal bandpass-gefiltert sind.

In einer weiteren bevorzugten Variante umfasst die spektrale Modulations-Bandpassfilterung in Schritt c) eine Tiefpassfilterung der spektro-temporalen Repräsentation mit Tiefpassfiltern mit monoton zunehmender spektraler Breite entlang der Signalfrequenzen und eine Differenzbildung zwischen einer tiefpass-gefilterten Repräsentation und der benachbarten tiefpass-gefilterten Repräsentation mit der nächsthöheren spektralen Breite.

Neben dem oben beschriebenen Verfahren betrifft die Erfindung eine Vorrichtung zur rechnergestützten Verarbeitung von Audiosignalen, wobei die Vorrichtung dazu eingerichtet ist, das erfindungsgemäße Verfahren bzw. eine oder mehrere bevorzugte Varianten des erfindungsgemäßen Verfahrens durchzuführen. Mit anderen Worten enthält diese Vorrichtung neben einer Einheit zum digitalen Erfassen der Audiosignale ein Rechnermittel, mit dem alle weiteren Schritte des erfindungsgemäßen Verfahrens bzw. bevorzugter Varianten dieses Verfahrens durchgeführt werden können.

In einer besonders bevorzugten Ausgestaltung ist die erfindungsgemäße Vorrichtung eine Hörhilfe, wie z.B. ein Hörgerät (d.h. ein Gerät, welches aus dem Ausgangssignal ein Schallsignal generiert) oder ein Cochlea-Implantat. Im Falle eines Hörgeräts ist in der Vorrichtung auch eine Einheit zur Wandlung der modifizierten Ausgangssignale in akustische Signale vorgesehen. Im Falle eines Cochlea-Implantats ist eine Einheit zur Wandlung der modifizierten Ausgangssignale in entsprechende Strompulse der Elektrodenanordnung des Implantats vorgesehen.

Ein Ausführungsbeispiel der Erfindung wird nachfolgend anhand der beigefügten Figuren detailliert beschrieben.

Es zeigen:

Fig. 1     ein Flussdiagramm, welches den Ablauf einer Ausführungsform des erfindungsgemäßen Verfahrens wiedergibt;

Fig. 2     ein Diagramm, welches die Übertragungsfunktionen von Bandpassfiltern zeigt, die in einer Ausführungsform der Erfindung zur Erzeugung von spektralen Zeitsignalen verwendet werden können;

Fig. 3 und Fig. 4     Diagramme, welche eine Kompressionsfunktion zur Dynamickompression gemäß einer Variante

EP 3 689 005 B1

der Erfindung wiedergeben.

Gemäß der nachfolgend beschriebenen Ausführungsform wird ein Eingangssignal A in der Form eines digital erfassten Audiosignals verarbeitet. Dieses Eingangssignal kann beispielsweise mittels eines Mikrofons mit Analog-Digital-Wandler erfasst worden sein. Die nachfolgend erläuterten Verfahrensschritte werden dabei durch eine geeignete Rechnereinheit durchgeführt. In einer bevorzugten Variante der Erfindung ist diese Rechnereinheit Bestandteil eines Hörgeräts, das über ein Mikrofon Audiosignale erfasst und nach deren Verarbeitung entsprechend modifizierte Audiosignale über einen Lautsprecher in den menschlichen Gehörgang einspeist. Die Erfindung kann jedoch auch für andere Einsatzzwecke verwendet werden, welche weiter unten noch genannt werden.

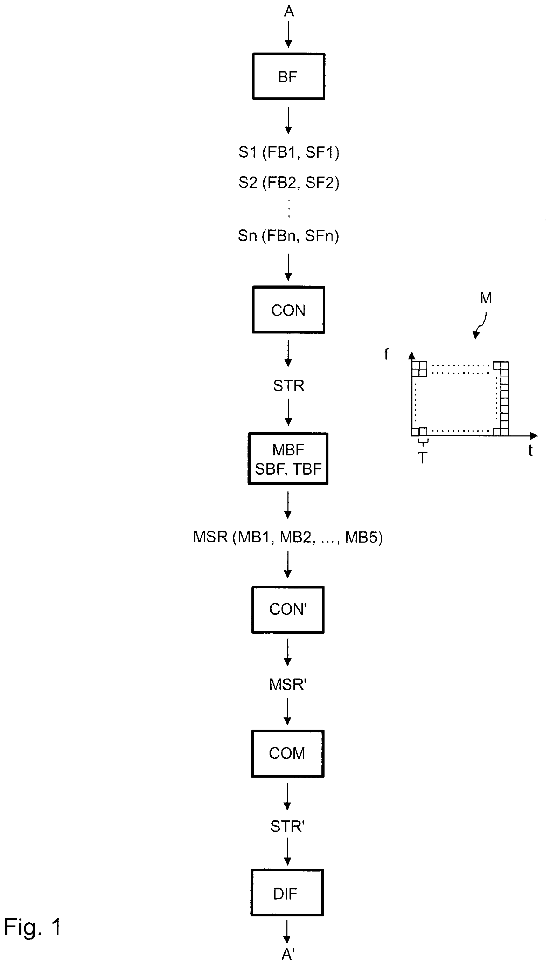

Gemäß dem Ablaufdiagramm der Fig. 1 wird das Eingangssignal A mittels einer Bandpassfilterung BF in spektrale Zeitsignale in einer Vielzahl von Frequenzbändern mit zugeordneten Mittenfrequenzen zerlegt. Im Folgenden werden diese Mittenfrequenzen auch als Signalfrequenzen bezeichnet. Man erhält somit entsprechende spektrale Zeitsignale S1, S2, ..., Sn, wobei n die Anzahl von Frequenzbändern repräsentiert. Das spektrale Zeitsignal S1 entspricht dabei dem Frequenzband FB1 mit der niedrigsten Mitten- bzw. Signalfrequenz SF1, das spektrale Zeitsignal S2 dem Frequenzband FB2 mit der zweithöchsten Signalfrequenz SF2 und so weiter. Das spektrale Zeitsignal Sn gehört somit zu dem Frequenzband FBn mit der höchsten Signalfrequenz SFn. Die einzelnen spektralen Zeitsignale bestehen aus einer Vielzahl von Signalpegeln zu aufeinander folgenden Zeitpunkten. In der hier beschriebenen Ausführungsform sind die Signalpegel die Pegel der Absolutwerte der Amplituden des jeweiligen spektralen Zeitsignals in dB SPL. Die Amplituden werden jedoch gespeichert, wobei am Ende des hier beschriebenen Verfahrens die Differenzen auf diese Amplituden der entsprechenden Zeitsignale S1 bis Sn angewendet werden.

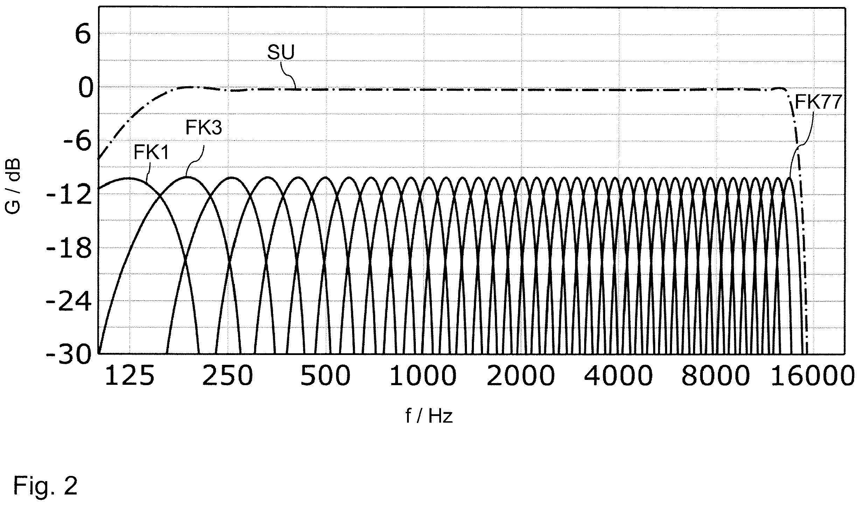

Fig. 2 zeigt ein Diagramm, welches die Übertragungsfunktionen von Bandpassfiltern verdeutlicht, die bei der Bandpassfilterung BF der Fig. 1 verwendet werden können. Die einzelnen Übertragungsfunktionen sind als Kurven mit durchgezogenen Linien dargestellt und fortlaufend nummeriert, wobei aus Übersichtlichkeitsgründen nur einige Übertragungsfunktionen mit Bezugszeichen FK1, FK3 bzw. FK77 bezeichnet sind. Die Übertragungsfunktionen geben den Gewinn G in dB in Abhängigkeit von der Frequenz f in Hz wieder. Es werden insgesamt 78 Übertragungsfunktionen und damit 78 Bandpassfilter verwendet, wobei in Fig. 2 aus Übersichtlichkeitsgründen nur jede zweite Übertragungsfunktion wiedergegeben ist. Darüber hinaus ist in Fig. 2 die Summe der Übertragungsfunktionen durch die strichpunktierte Linie SU angedeutet. Die Summe der Übertragungsfunktionen weicht im Bereich von 200 Hz bis über 12 kHz weniger als 0,5 dB von der idealen Linie bei 0 dB ab und ist damit auch ggf. für die Verarbeitung von HiFi-Signalen geeignet.

Anstatt der in Fig. 2 gezeigten Bandpassfilter können ggf. auch andere Bandpassfilter aus entsprechenden Filterbanken genutzt werden, wie z.B. aus der an sich bekannten Gammaton-Filterbank. Generell sollte die spektrale Zerlegung derart gewählt werden, dass deren zeitliche und spektrale Auflösung in etwa dem menschlichen Gehör entspricht und eine Resynthese des Eingangssignals ohne wahrnehmbare Signalverzerrungen durch Addition der spektralen Zeitsignale möglich ist. Vorzugsweise sollte dabei der Abstand zwischen den Mittenfrequenzen von benachbarten Filtern höchstens 1,0 ERB, bevorzugt jedoch höchstens 0,5 ERB, betragen.

Die aus der Bandpassfilterung BF erhaltenen spektralen Zeitsignale werden in einem nächsten Schritt CON in eine spektro-temporale Repräsentation STR konvertiert. Dabei werden die einzelnen spektralen Zeitsignale S1 bis Sn zunächst mit einer geeigneten Fensterlänge im Zeitbereich, z.B. 1 ms, gefenstert. Für jedes Zeitfenster wird das Maximum des Signalpegels eines jeweiligen spektralen Zeitsignals innerhalb des Fensters bestimmt. Dieses Maximum wird als einzelner Signalpegel dem entsprechenden Zeitfenster zugeordnet. Mittels der Fensterung kann eine Rechenzeit- und Datenreduktion erreicht werden.

Auf die gefensterten spektralen Zeitsignale, welche nur noch Signalpegel für entsprechende Zeitfenster enthalten, werden anschließend rudimentäre Maskierungseffekte angewendet, um einige Signaleigenschaften, die Normalhörende nicht wahrnehmen können, zu entfernen. In der hier beschriebenen Ausführungsform wird sowohl eine temporale als auch eine spektrale Maskierung verwendet.

Im Rahmen der temporalen Maskierung wird der Signalpegel jedes spektralen Zeitsignals über einen Zeitabschnitt aus mehreren Zeitfenstern (z.B. 15 ms) konstant gehalten, solange sich der Signalpegel in diesem Zeitabschnitt nicht erhöht. Im Falle, dass sich der Signalpegel innerhalb des Zeitabschnitts erhöht, wird der erhöhte Signalpegel in einem neu beginnenden Zeitabschnitt weiterverwendet. Sollte sich der Signalpegel nicht erhöhen und damit im gesamten Zeitabschnitt konstant bleiben, wird er danach mit 1 dB pro Zeitfenster erniedrigt, bis der (ursprüngliche) Signalpegel für ein Zeitfenster höher als der erniedrigte Signalpegel wird, woraufhin der höhere Signalpegel für das entsprechende Zeitfenster weiterverwendet wird und ein neuer Zeitabschnitt begonnen wird. Mit dieser temporalen Maskierung bleiben schnelle Anstiege der Signalpegel erhalten, während schnelle Abfälle des Signalpegels nicht mehr dargestellt sind. Mittels der temporalen Maskierung kann eine Verbesserung der Verständlichkeit von Sprachsignalen erreicht werden.

Nach der temporalen Maskierung wird die spektrale Maskierung durchgeführt, bei der die spektralen Signale in einem jeweiligen Zeitfenster für alle Frequenzbänder betrachtet werden. In dem entsprechenden Zeitfenster wird dabei eine Verarbeitung für jeden Signalpegel des entsprechenden spektralen Signals durchgeführt, wobei hierfür für jeden Signalpegel eine Kopie aller Signalpegel im entsprechenden Zeitfenster erstellt und in einem Zwischenspeicher abgelegt wird. In der jeweiligen Kopie werden Nachbarsignalpegel berücksichtigt, welche spektral (d.h. in Richtung der Signal- bzw. Mittenfrequenzen) zu dem gerade betrachteten Signalpegel benachbart sind. Die Nachbarsignalpegel werden dabei mit entsprechenden Abschwächungsfaktoren abgeschwächt bzw. gedämpft, wobei die Abschwächung umso größer wird, je größer der spektrale Abstand des entsprechenden Nachbarsignalpegels vom gerade betrachteten Signalpegel ist. Ein Beispiel von Abschwächungsfaktoren ist in der nachfolgenden Tabelle wiedergegeben, wobei die Position 0 dem gerade betrachteten Signalpegel entspricht.

| Distanz in ERB | -8 | -4 | -2 | -1 | 0 | 1 | 2 | 4 | 8 |
|---|---|---|---|---|---|---|---|---|---|
| Abschwächung in dB | -80 | -60 | -30 | -10 | 0 | -20 | -60 | -120 | -160 |

Wie man aus dieser Tabelle erkennt, wird beispielsweise der Nachbarsignalwert, der eine spektrale Position um 1 ERB höher als der betrachtete Signalwert hat, um 20 dB reduziert. Demgegenüber wird der Nachbarsignalwert, dessen spektrale Position um 1 ERB niedriger als der betrachtete Signalwert ist, um nur 10 dB reduziert.

Wird nach Durchführung der Dämpfung festgestellt, dass der Maximalwert der gedämpften Nachbarsignalpegel größer als der gerade betrachtete Signalpegel ist, ersetzt dieser Maximalwert den gerade betrachteten Signalpegel. Ist das nicht der Fall, wird der gerade betrachtete Signalpegel unverändert gelassen. Vorzugsweise werden alle Signalpegel, die danach noch unterhalb der Ruhehörschwelle eines Normalhörenden liegen, auf den Wert dieser Ruhehörschwelle gesetzt. Mittels der spektralen Maskierung kann erreicht werden, dass Töne, die Normalhörende nicht hören, weniger stark verstärkt werden.

Nach Durchführung der Schritte der Fensterung sowie der temporalen und spektralen Maskierung erhält man schließlich die spektro-temporale Repräsentation STR des ursprünglichen Eingangssignals A. Diese spektro-temporale Repräsentation ist in Fig. 1 durch eine Matrix M veranschaulicht. Die einzelnen Einträge der Matrix sind durch kleine Rechtecke angewendet und stellen jeweils einen Signalpegel dar. Die Erstreckung der jeweiligen Zeilen der Matrix M entspricht der Zeitachse t und die Erstreckung der jeweiligen Spalten der Frequenzachse f. Mit anderen Worten repräsentiert jede Spalte ein Zeitfenster T, wohingegen jede Zeile für eine Signalfrequenz bzw. Mittenfrequenz steht. Ein jeweiliger Eintrag der Matrix ist somit einem Frequenzband und einem Zeitfenster zugeordnet.

In einem nächsten Schritt wird die spektro-temporale Repräsentation STR einer Modulations-Bandpassfilterung MBF unterzogen, die eine spektrale Modulations-Bandpassfilterung SPF und eine temporale Modulations-Bandpassfilterung TBF enthält. Bei der spektralen Modulations-Bandpassfilterung macht man sich die Erkenntnis zunutze, dass spektrale Strukturen, insbesondere in einer Größenordnung um 2 bis 4 ERB, für die Sprachverständlichkeit besonders wichtig sind, wohingegen temporale Strukturen entgegen weit verbreiteter Meinungen eine untergeordnete Rolle spielen.

Zur Umsetzung der spektralen und temporalen Modulations-Bandpassfilterung wird die spektro-temporale Repräsentation STR zunächst in mehreren Schichten mit sinkenden Abschnittsfrequenzen spektro-temporal (d.h. 2-dimensional) tiefpass-gefiltert (d.h. geglättet). Für die Tiefpassfilterung wurden in der hier beschriebenen Ausführungsform Glättungsfilter mit Hanning-Fenster mit monoton steigenden Breiten verwendet. Mit anderen Worten wird eine Faltung der spektro-temporalen Repräsentation STR mit einer 2-dimensionalen Hanning-Fensterfunktion der entsprechenden Breite durchgeführt. Anschließend werden die Differenzen der jeweils benachbarten Schichten gebildet. In der hier beschriebenen Ausführungsform erfolgt die Tiefpassfilterung in sechs Schichten S1 bis S6, welche in der nachfolgenden Tabelle angegeben sind:

| Schicht | S1 | S2 | S3 | S4 | S5 | S6 |
|---|---|---|---|---|---|---|
| Spektrale Breite in ERB | 0,5 | 2 | 4 | 4 | 8 | 16 |
| Temporale Breite in ms | 1 | 1 | 1 | 2 | 2 | 2 |

Im Folgenden werden Frequenzen der spektralen Modulations-Bandpassfilterung als spektrale Modulationsfrequenzen bzw. einfach auch als Modulationsfrequenzen bezeichnet. Demgegenüber werden Frequenzen der temporalen Modulations-Bandpassfilterung temporale Modulationsfrequenzen genannt.

Gemäß obiger Tabelle werden in der Schicht S1 Signale mit einer spektralen Breite von 0,5 ERB extrahiert, was einer Extraktion von Modulationsfrequenzen bis maximal 2/ERB entspricht. In der Schicht S2 werden entsprechend der spektralen Breite von 2 ERB Modulationsfrequenzen bis maximal 0,5/ERB extrahiert. In der Schicht S3 werden entsprechend der spektralen Breite von 4 ERB Modulationsfrequenzen von maximal 0,25/ERB gewonnen. In den Schichten S1 bis S3 wurde ferner eine temporale Tiefpassfilterung mit einer temporalen Breite von 1 ms (d.h. bis zu einer temporalen Modulationsfrequenz von 1000 Hz) durchgeführt. Die Schicht S4 entspricht der Schicht S3, wobei jedoch eine temporale Tiefpassfilterung mit der temporalen Breite von 2 ms und damit bis zu einer temporalen Modulationsfrequenz von maximal 500 Hz durchgeführt wird. Die Schicht S5 extrahiert entsprechend der spektralen Breite von 8 ERB Signalanteile bis zu einer maximalen spektralen Modulationsfrequenz von 0,125/ERB bei Beibehaltung der temporalen Tiefpassfilterung mit der temporalen Breite von 2 ms. Die Schicht S6 extrahiert entsprechend der spektralen Breite von 16 ERB Signalanteile mit einer spektralen Modulationsfrequenz bis maximal 0,0625/ERB bei Beibehaltung der temporalen Tiefpassfilterung mit der temporalen Breite von 2 ms.

Um aus den tiefpass-gefilterten Signalen der Schichten S1 bis S6 bandpass-gefilterte Signale zu gewinnen, werden die Differenzen zwischen den einzelnen Schichten ermittelt. Mit anderen Worten werden folgende Differenzen bestimmt:

- die Differenz D1 zwischen der Schicht S1 und S2, d.h. D1=S1-S2;
- die Differenz D2 zwischen der Schicht S2 und der Schicht S3, d.h. D2 = S2-S3;
- die Differenz D3 zwischen der Schicht S3 und der Schicht S4, d.h. D3 = S3-S4;
- die Differenz D4 zwischen der Schicht S4 und der Schicht S5, d.h. D4 = S4-S5;
- die Differenz D5 zwischen der Schicht S5 und der Schicht S6, d.h. D5 = S5-S6;
- die Differenz D6 zwischen der Schicht S6 und 0, was der Schicht S6 entspricht.

Die obige Wahl der Differenzen führt dazu, dass durch die Summation aller Differenzen D1 bis D6 wieder die ursprüngliche spektro-temporale Repräsentation gewonnen werden kann.

Alle Differenzen D1 bis D6 entsprechen spektral bandpass-gefilterten Signalen, wobei das Signal D3 zusätzlich temporal bandpass-gefiltert ist. Diese Signale stellen erste Modulationssignal-Repräsentationen im Sinne der Patentansprüche dar und sind in Fig. 1 allgemein mit dem Bezugszeichen MSR bezeichnet. Das Format dieser Repräsentationen sowie auch der weiter unten erläuterten Repräsentation MSR' entspricht der Matrix M aus Fig. 1. Das Signal gemäß der Differenz D1 entspricht einer spektralen Modulations-Bandpassfilterung im spektralen Modulationsfrequenzband MB1 zwischen 2/ERB und 0,5/ERB (entsprechend den Kehrwerten der in obiger Tabelle genannten spektralen Breiten). Das Signal gemäß der Differenz D2 entspricht einer spektralen Modulations-Bandpassfilterung im spektralen Modulationsfrequenzband MB2 zwischen 0,5/ERB und 0,25/ERB. Das Signal gemäß der Differenz D3 entspricht einer spektralen Modulations-Bandpassfilterung in gleichen Modulationsfrequenzband MB2 wie das Signal gemäß der Differenz D2, jedoch mit einer zusätzlichen temporalen Modulations-Bandpassfilterung. Das Signal gemäß der Differenz D4 entspricht einer spektralen Modulations-Bandpassfilterung im spektralen Modulationsfrequenzband MB3 zwischen 0,25/ERB und 0,125/ERB. Das Signal gemäß der Differenz D5 entspricht einer spektralen Modulations-Bandpassfilterung im spektralen Modulationsfrequenzband MB4 zwischen 0,125/ERB und 0,0625/ERB. Das Signal gemäß der Differenz D6 entspricht einer Modulations-Bandpassfilterung im spektralen Modulationsfrequenzband MB5 zwischen 0,0625/ERB und 0/ERBs.

Basierend auf den Modulationssignal-Repräsentationen MSR wird eine modifizierte spektro-temporale Repräsentation bestimmt, wobei eine Kompression unter Bewahrung der essentiellen Sprachmodulationen durchgeführt wird. Dabei macht man sich die Erkenntnis zunutze, dass schmale (d.h. hochfrequente) spektrale Strukturen von kleiner 4 ERB besonders kritisch für eine gute Spracherkennung sind. Deshalb werden diese Strukturen erhalten. Demgegenüber werden breitbandige Strukturen von größer 4 ERB komprimiert. Nur sehr kurze temporale Strukturen von kleiner 2 ms werden erhalten, während längere spektrale Strukturen von größer als 2 ms komprimiert werden. Im Folgenden wird erläutert, wie die modifizierte spektro-temporale Repräsentation basierend auf den bandpass-gefilterten Differenzsignalen D1 bis D6 bestimmt werden kann.

Die einzelnen Modulationssignal-Repräsentationen MSR, die den Differenzsignalen D1 bis D6 entsprechen, werden in dem Konvertierungsschritt CON' der Fig. 1 zunächst geeignet manipuliert, so dass manipulierte Modulationssignal-Repräsentationen MSR' erhalten werden. Dabei werden die Differenzen D1 bis D5 durch die Multiplikation mit entsprechenden Faktoren F1 bis F5 verändert, wohingegen auf die Differenz D6 eine Kompressionsfunktion k zur Verminderung des Dynamikbereichs angewendet wird. Die manipulierten Signale werden anschließend gemäß dem Schritt COM der Fig. 1 summiert, wodurch die modifizierte spektro-temporale Repräsentation STR' erhalten wird. Mit anderen Worten lautet die modifizierte spektro-temporale Repräsentation wie folgt:

$$F1{\cdot}D1+F2{\cdot}D2+F3{\cdot}D3+F4{\cdot}D4+F5{\cdot}D5+k(D6)$$

In der hier beschriebenen Ausführungsform wird F1 = 1 gewählt, wodurch die schmalsten spektralen Strukturen erhalten bleiben. Des Weiteren wird F2 $\geq$ 1 gewählt, um die für die Spracherkennung wichtigen spektralen Strukturen zu erhalten bzw. ggf. auch zu expandieren, um beispielsweise mögliche überschwellige Hörverluste zu kompensieren. Die weiteren Faktoren F3 bis F5 werden vorzugsweise auch auf 1 gesetzt, um die Qualität des Signals so gut wie möglich zu erhalten. Das Signal k(D6) ist ein in der Dynamik komprimiertes Signal, was über die Kompressionsfunktion k erreicht wird, wie weiter unten noch näher erläutert wird.

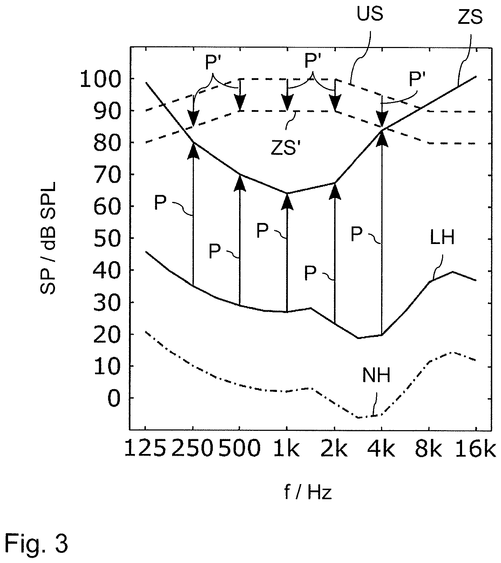

Die obige Summe umfasst auf jeden Fall die Signalanteile F1·D1, F2·D2 sowie k(D6). Da jede weitere der Differenzen D3 bis D5 mehr Dynamik im Ausgangssignal erzeugt, werden die entsprechenden Terme F3·D3, F4·D4 und F5·D5 schrittweise in dieser Reihenfolge immer nur dann hinzugefügt, wenn im jeweiligen Zeitfenster eine Verfügbarkeitsbedingung erfüllt ist. Verfügbar bedeutet dabei, dass durch das Hinzufügen eines Terms, der sowohl positiv als auch negativ sein kann, keine Signalanteile außerhalb eines vorgegebenen Zielbereichs der Signalpegel liegen. Sollte demzufolge die Hinzufügung eines Terms dazu führen, dass der Signalpegel für zumindest eine Signalfrequenz im entsprechenden Zeitfenster außerhalb des Zielbereichs liegt, wird dieser Term und auch die weiteren Terme (sofern vorhanden) nicht mehr hinzuaddiert. In der hier beschriebenen Ausführungsform erstreckt sich der Zielbereich gemäß der weiter unten erläuterten Fig. 3 zwischen einer unteren Zielhörschwelle ZS, welche der Ruhehörschwelle eines Schwerhörenden entspricht, und einer oberen Zielhörschwelle ZS', welche der Unbehaglichkeitsschwelle eines Schwerhörenden entspricht. Die Schwellen ZS und ZS' sind frequenzabhängig, d.h. sie hängen von den entsprechenden Signalfrequenzen im verarbeiteten Signal ab.

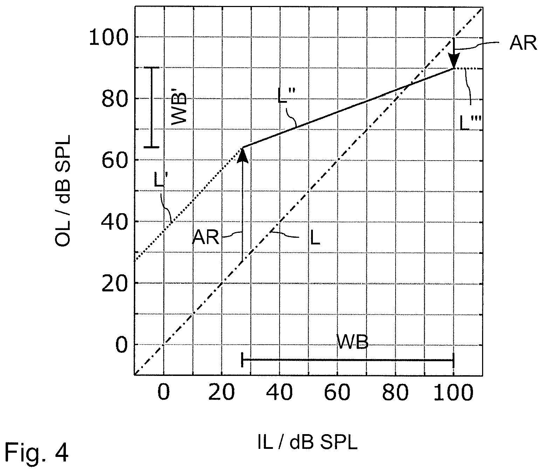

Im Folgenden wird anhand von Fig. 3 und Fig. 4 erläutert, wie die Kompressionsfunktion k in der hier beschriebenen Ausführungsform festgelegt wird. Fig. 3 zeigt dabei, wie in Abhängigkeit von der Signalfrequenz f eines entsprechenden Signalpegels SP der Differenz D6 bestimmte Hörschwellen aufeinander abgebildet werden. Entlang der Abszisse der Fig. 3 ist die Frequenz f in Hz und entlang der Ordinate der Signalpegel SP in dB SPL angegeben. Die strichpunktierte Linie NH gibt die Ruhehörschwelle eines Normalhörenden an. Demgegenüber entspricht die untere durchgezogene Linie LH einer geeignet gewählten Hörschwelle eines Normalhörenden für leise Signale, welche durch Verschiebung der Ruhehörschwelle NH um einen vorbestimmten Signalpegel nach oben erhalten wird. Die ebenfalls als durchgezogene Linie gezeigte Hörschwelle ZS ist die bereits oben genannte Ruhehörschwelle eines Schwerhörenden. Demgegenüber bezeichnet die gestrichelte Linie US die Unbehaglichkeitsschwelle US eines Normalhörenden. Die darunter liegende gestrichelte Linie ZS' entspricht der ebenfalls bereits oben erwähnten Unbehaglichkeitsschwelle eines Schwerhörenden. Gemäß der Funktion k(D6) wird nunmehr erreicht, dass die Hörschwelle LH auf die Ruhehörschwelle ZS eines Schwerhörenden abgebildet wird, was durch die Pfeile P angedeutet ist. In gleicher Weise wird erreicht, dass die Unbehaglichkeitsschwelle US eines Normalhörenden auf die Unbehaglichkeitsschwelle ZS' eines Schwerhörenden abgebildet wird, wie durch die Pfeile P' angedeutet ist. Es wird somit eine Dynamikkompression erreicht, um eine geeignete Wahrnehmung von akustischen Signalen für einen Schwerhörenden zu ermöglichen.

Fig. 4 verdeutlicht nochmals für eine Signalfrequenz von 1000 Hz die Wandlung des Eingangspegels IL des Differenzsignals D6 (Abszisse in dem Diagramm der Fig. 4) in einen entsprechenden Ausgangspegel OL (Ordinate in dem Diagramm der Fig. 4). Durch die strichpunktierte Linie L, die der Winkelhalbierenden zwischen der Abszisse und Ordinate entspricht, wird der Fall angedeutet, dass keine Signalveränderung erfolgt. Der Verlauf der Funktion k zur Dynamikkompression ist durch die gepunktete Linie L', die durchgezogene Linie L" sowie die weitere gepunktete Linie L‴ angedeutet. Der Wertebereich WB, welcher der Differenz zwischen der Schwelle LH und der Schwelle US bei 1000 Hz gemäß Fig. 3 entspricht, wird gemäß der Funktion k auf den verkleinerten Wertebereich WB' abgebildet, welcher der Differenz zwischen der Schwelle ZS und der Schwelle ZS' bei 1000 Hz gemäß Fig. 3 entspricht. Dies wird in Fig. 4 auch nochmals durch die Pfeile AR verdeutlicht.

Wie man aus Fig. 4 ferner erkennt, wird der Signalpegelbereich bis zu Beginn des Wertebereichs WB (d.h. bis zur Schwelle LH) linear abgebildet, was durch die Linie L' deutlich wird. Ferner wird der Signalpegelbereich rechts neben dem Wertebereich WB, der oberhalb der Unbehaglichkeitsschwelle US eines Normalhörenden liegt, immer auf die Unbehaglichkeitsschwelle ZS' eines Schwerhörenden abgebildet, was durch die Linie L‴ ersichtlich wird. Zwischen den Schwellen LH und US erfolgt die Dynamikkompression gemäß der Linie L" der Fig. 4.

Nach der Kombination der Signale gemäß der obigen Summation der Differenzen erhält man die modifizierte spektro-temporale Repräsentation STR', die das gewünschte Ausgangssignal beschreibt. Um das Ausgangssignal zu generieren, wird gemäß dem Schritt DIF der Fig. 1 die Differenz zwischen der modifizierten spektro-temporalen Repräsentation STR' und der ursprünglichen spektro-temporalen Repräsentation STR gebildet. Hieraus ergeben sich Verstärkungsfaktoren, die dann auf die spektralen Zeitsignale S1, S2, ..., Sn angewendet werden. Da diese Zeitsignale noch nicht gefenstert sind und demzufolge mehr Zeitpunkte als Zeitfenster enthalten, wird der Signalwert an dem Zeitpunkt in der Mitte eines jeweiligen Zeitfensters auf den mit dem entsprechenden Verstärkungsfaktor des Zeitfensters verstärkten Signalpegel gesetzt und die Werte für Zeitpunkte zwischen benachbarten Zeitfenstern werden geeignet interpoliert. Nach Anwendung der Differenzen auf die entsprechenden Zeitsignale S1 bis Sn erhält man schließlich durch deren Summation das modifizierte Ausgangssignal A', das bei der Verwendung des Verfahrens in einem Hörgerät nach einer Digital-Analog-Wandlung über einen entsprechenden Lautsprecher ausgegeben werden kann.

Die obige Implementierung des erfindungsgemäßen Verfahrens wurde von den Erfindern im Rahmen von Versuchen mit Schwerhörenden getestet. Dabei hat sich ergeben, dass in einer Reihe von Fällen eine deutliche Verbesserung der Verständlichkeit von Sprache sowohl in Ruhe als auch mit Störgeräusch im Vergleich zu bekannten Verfahren zur Dynamikkompression erreicht werden kann. Im Besonderen wird eine Verbesserung der Sprachverständlichkeit bei einem fluktuierenden Störgeräusch erreicht.

Das erfindungsgemäße Verfahren kann in einer Vielzahl von unterschiedlichen Anwendungen zum Einsatz kommen. Das Verfahren kann in Hörgeräten und Cochlea-Implantaten verwendet werden, um hierdurch die Verständlichkeit von Sprache für einen Schwerhörenden zu verbessern. Ein weiterer Einsatzbereich des erfindungsgemäßen Verfahrens ist in Telekommunikations-, Entertainment- und Infotainment-Systemen, um die Dynamik der Wiedergabe von Sprachsignalen und gegebenenfalls auch von Musik adaptiv an die akustische Situation anzupassen, wie z.B. an eine reduzierte verfügbare Dynamik durch Fahrgeräusche in einer Fahrkabine. Ein weiterer Anwendungsfall ist die Pegelnormalisierung bei Konferenzschaltungen, bei der jeder Teilnehmer unabhängig von dem Pegel, der Färbung und der Dynamik des Eingangssignals nur eine festgelegte Ausgangsdynamik zugewiesen bekommt.

**Patentansprüche**

1. Verfahren zur rechnergestützten Verarbeitung von Audiosignalen, bei dem:

   a) als Eingangssignal (A) ein Audiosignal in der Form eines Zeitsignals aus Amplitudenwerten digital erfasst wird und die im Eingangssignal (A) enthaltenen spektralen Zeitsignale (S1, S2, ..., Sn) in mehreren Frequenzbändern (FB1, FB2, ..., FBn) mit zugeordneten Signalfrequenzen (SF1, SF2, ..., SFn) in der Form Mittenfrequenzen der jeweiligen Frequenzbänder (FB1, FB2, ..., FBn) bestimmt werden, wodurch eine Vielzahl von Signalpegeln für jedes spektrale Zeitsignal (S1, S2, ..., Sn) erhalten werden;
   b) aus den spektralen Zeitsignalen (S1, S2, ..., Sn) eine spektro-temporale Repräsentation (STR) des Eingangssignals (A) ermittelt wird, die für jeweilige Zeitfenster (T) eine Vielzahl von Signalpegeln enthält, wobei die Signalpegel in einem Zeitfenster (T) jeweils einer anderen Signalfrequenz (SF1, SF2, ..., SFn) zugeordnet sind;
   c) die spektro-temporale Repräsentation (STR) einer Modulations-Bandpassfilterung (MBF) umfassend eine spektrale Modulations-Bandpassfilterung (SBF) unterzogen wird, wobei durch die spektrale Modulations-Bandpassfilterung (SBF) aus mehreren nicht überlappenden spektralen Modulationsfrequenzbändern (MB1, MB2, .., MB5), denen jeweils ein Frequenzwert in der Form der minimalen spektralen Modulationsfrequenz im entsprechenden Modulationsfrequenzband (MB1, MB2, .., MB5) zugeordnet ist, jeweils die spektrale Variation der im jeweiligen Modulationsfrequenzband (MB1, MB2, .., MB6) enthaltenen Signalanteile entlang der Signalfrequenzen (SF1, SF2, ..., SFn) extrahiert wird, wodurch für jedes Modulationsfrequenzband (MB1, MB2, .., MB5) eine oder mehrere erste Modulationssignal-Repräsentationen (MSR) erhalten werden, wobei jede erste Modulationssignal-Repräsentation (MSR) für jeweilige Zeitfenster (T) eine Vielzahl von Modulationssignalpegeln enthält, wobei die Modulationssignalpegel in einem Zeitfenster (T) jeweils einer anderen Signalfrequenz (SF1, SF2, ..., SFn) zugeordnet sind;
   d) die ersten Modulationssignal-Repräsentationen (MSR) in zweite Modulationssignal-Repräsentationen (MSR') gewandelt werden, wobei zumindest ein Teil der zweiten Modulationssignal-Repräsentationen (MSR) gegenüber der entsprechenden ersten Modulationssignal-Repräsentation (MSR) modifiziert ist, wobei für das Modulationsfrequenzband (MB5) mit dem niedrigsten Frequenzwert die erste Modulationssignal-Repräsentation (MSR) mittels einer Kompressions-Funktion in die zweite Modulationssignal-Repräsentation (MSR') derart gewandelt wird, dass für jede Signalfrequenz (SF1, SF2, ..., SFn) in einem jeweiligen Zeitfenster (T) ein vorgegebener Wertebereich (WB) der Modulationssignalpegel der ersten Modulationssignal-Repräsentation (MSR) auf einen verkleinerten Wertebereich (WB') der Modulationssignalpegel der zweiten Modulationssignal-Repräsentation (MSR') abgebildet wird, und wobei zumindest eine weitere zweite Modulationssignal-Repräsentation (MSR') durch Multiplikation der entsprechenden ersten Modulationssignal-Repräsentation (MSR) mit einem Faktor größer Null erhalten wird;
   e) eine modifizierte spektro-temporale Repräsentation (STR') ermittelt wird, indem die zweiten Modulationssignal-Repräsentationen (MSR') derart kombiniert werden, dass für ein jeweiliges Zeitfenster (T) jeder Signalpegel, der in der unmodifizierten spektro-temporalen Repräsentation (STR) oberhalb einer vorgegebenen Hörschwelle liegt, in der modifizierten spektro-temporalen Repräsentation (STR') auf einen Signalpegel innerhalb eines Zielpegelbereichs abgebildet wird, wobei der Zielpegelbereich von der Signalfrequenz (SF1, SF2, ..., SFn) abhängt und durch eine untere Zielhörschwelle (ZS) nach unten begrenzt ist;
   f) aus der modifizierten spektro-temporalen Repräsentation (STR') ein Ausgangssignal (A') erzeugt wird, das gegenüber dem Eingangssignal (A) modifiziert ist.

2. Verfahren nach Anspruch 1, wobei der Zielpegelbereich ferner durch eine obere Zielhörschwelle (ZS') nach oben begrenzt ist.

3. Verfahren nach Anspruch 1 oder 2, wobei die Wandlung der ersten Modulationssignal-Repräsentationen (MSR) in zweite Modulationssignal-Repräsentationen (MSR') in Schritt d) derart durchgeführt wird, dass alle zweiten Modulationssignal-Repräsentationen (MSR'), deren jeweiliges Modulationsfrequenzband (MB1, MB2, ..., MB5) Modulationsfrequenzen von größer gleich $0,25/ERB$ enthält, gegenüber den entsprechenden ersten Modulationssignal-Repräsentationen (MSR) unmodifiziert bleiben oder verstärkt werden.

4. Verfahren nach einem der vorhergehenden Ansprüche, wobei die Wandlung der ersten Modulationssignal-Repräsentationen (MSR) in zweite Modulationssignal-Repräsentationen (MSR') in Schritt d) derart durchgeführt wird, dass für das Modulationsfrequenzband (MB1) mit dem höchsten Frequenzwert jede zweite Modulationssignal-Repräsentation (MSR') mit der ersten Modulationssignal-Repräsentation (MSR) identisch ist und eine oder mehrere zweite Modulationssignal-Repräsentationen (MSR') umfassend zumindest eine zweite Modulationssignal-Repräsentation (MSR') für das Modulationsfrequenzband (MB2) mit dem zweithöchsten Frequenzwert und/oder jede zweite Modulationssignal-Repräsentation (MSR') für das Modulationsfrequenzband (MB5) mit dem niedrigsten Frequenzwert gegenüber der entsprechenden ersten Modulationssignal-Repräsentation (MSR) modifiziert ist.

5. Verfahren nach einem der vorhergehenden Ansprüche, wobei die zweiten Modulationssignal-Repräsentationen (MSR') in Schritt e) derart kombiniert werden, dass zu der oder den zweiten Modulationssignal-Repräsentationen (MSR') für das Modulationsfrequenzband (MB5) mit dem niedrigsten Frequenzwert zumindest eine zweite Modulationssignal-Repräsentation (MSR') für das Modulationsfrequenzband (MB2) mit dem zweithöchsten Frequenzwert und alle zweiten Modulationssignal-Repräsentationen (MSR') für das Modulationsfrequenzband (MB1) mit dem höchsten Frequenzwert hinzuaddiert werden und anschließend schrittweise die restlichen zweiten Modulationssignal-Repräsentationen (MSR') in der Reihenfolge hin zu Modulationsfrequenzbändern (MB1, MB2, ..., MB5) mit niedrigen Frequenzwerten hinzuaddiert werden, wobei vor dem Hinzuaddieren einer vorgegebenen Anzahl von Modulationssignalpegeln einer zweiten Modulationssignal-Repräsentation (MSR') aus den restlichen zweiten Modulationssignal-Repräsentationen (MSR') für ein jeweiliges Zeitfenster (T) überprüft wird, ob eine Zulässigkeitsbedingung für die vorgegebene Anzahl von Modulationssignalpegeln verletzt ist, wobei das Hinzuaddieren der vorgegebenen Anzahl für das jeweilige Zeitfenster (T) bei Verletzen der Zulässigkeitsbedingung beendet wird, wobei die Zulässigkeitsbedingung dann verletzt ist, wenn das Hinzuaddieren des Modulationssignalpegels in dem jeweiligen Zeitfenster (T) für zumindest eine Signalfrequenz (SF1, SF2, ..., SFn) in der vorgegebenen Anzahl zu einem Signalpegel außerhalb des Zielpegelbereichs führt, welcher vor dem Hinzufügen noch innerhalb des Zielpegelbereichs lag.

6. Verfahren nach einem der vorhergehenden Ansprüche, wobei der vorgegebene Wertebereich (WB) nach unten durch eine untere Hörschwelle begrenzt ist, welche vorzugsweise die Ruhehörschwelle (NH) eines Normalhörenden oder eine darüber liegende Hörschwelle (LH) ist, und der verkleinerte Wertebereich (WB') nach unten durch eine untere Hörschwelle begrenzt ist, welche vorzugsweise die Ruhehörschwelle (ZS) eines Schwerhörenden ist, wobei der vorgegebene Wertebereich (WB) und der verkleinerte Wertebereich (WB') vorzugsweise auch durch eine obere Hörschwelle nach oben begrenzt sind, wobei die obere Hörschwelle für den vorgegebenen Wertebereich (WB) vorzugsweise eine Unbehaglichkeitsschwelle (US) eines Normalhörenden ist und die obere Hörschwelle für den verkleinerten Wertebereich (WB) vorzugsweise eine Unbehaglichkeitsschwelle (ZS') eines Schwerhörenden ist.

7. Verfahren nach einem der vorhergehenden Ansprüche, wobei in Schritt b) die Signalpegel eines jeweiligen spektralen Zeitsignals (S1, S2, ..., Sn) derart verändert werden, dass die Signalpegel innerhalb eines jeweiligen Zeitfensters (T) durch einen Wert zwischen einschließlich dem Maximalwert und einschließlich dem Minimalwert der Signalpegel innerhalb des jeweiligen Zeitfensters (T), vorzugsweise durch den Maximalwert, ersetzt werden, wodurch einem Zeitfenster (T) ein einzelner Signalpegel eines jeweiligen spektralen Zeitsignals (S1, S2, ..., Sn) zugeordnet wird.

8. Verfahren nach Anspruch 7, wobei in Schritt b) die einzelnen Signalpegel der jeweiligen spektralen Zeitsignale (S1, S2, ..., Sn) für die jeweiligen Zeitfenster (T) derart angepasst werden, dass der ursprüngliche Signalpegel eines jeweiligen spektralen Zeitsignals (S1, S2, ..., Sn) über eine vorbestimmte Anzahl von mehreren Zeitfenstern (T) konstant gehalten wird und hierdurch aktualisierte ursprüngliche Signalpegel erzeugt werden, solange sich der ursprüngliche Signalpegel in der vorbestimmten Anzahl von mehreren Zeitfenstern (T) nicht erhöht, wobei im Falle, dass sich der ursprüngliche Signalpegel für ein Zeitfenster (T) in der vorbestimmten Anzahl von mehreren Zeitfenstern (T) erhöht, der erhöhte Signalpegel für das Zeitfenster (T) als aktualisierter ursprünglicher Signalpegel weiterverwendet wird, und wobei im Falle, dass sich der ursprüngliche Signalpegel in der vorbestimmten Anzahl von mehreren Zeitfenstern (T) nicht erhöht, der ursprüngliche Signalpegel in den Zeitfenstern (T), die auf die vorbestimmte Anzahl von mehreren Zeitfenstern (T) folgen, sukzessive erniedrigt wird und hierdurch aktualisierte ursprüngliche Signalpegel erzeugt werden, bis der nächste ursprüngliche Signalpegel für ein Zeitfenster (T) höher wird als der erniedrigte Signalpegel, woraufhin der höhere Signalpegel für das Zeitfenster (T) als aktualisierter ursprünglicher Signalpegel weiterverwendet wird.

9. Verfahren nach Anspruch 7 oder 8, wobei in Schritt b) in einem jeweiligen Zeitfenster (T) für jeden einzelnen Signalpegel der spektralen Zeitsignale (S1, S2, ..., Sn) in einer Kopie aller Signalpegel des jeweiligen Zeitfensters (T) eine vorbestimmte Anzahl von Signalpegeln, welche spektral gemäß der Reihenfolge der Signalfrequenzen (SF1, SF2, ..., SFn) zu dem jeweiligen einzelnen Signalpegel benachbart sind, gedämpft wird, wobei die Dämpfung umso höher ist, je größer der spektrale Abstand eines Signalpegels aus der vorbestimmte Anzahl von Signalpegeln zu dem jeweiligen einzelnen Signalpegel gemäß der Reihenfolge der Signalfrequenzen (SF1, SF2, ..., SFn) ist, wobei im Falle, dass der Maximalwert der gedämpften Signalpegel der vorbestimmten Anzahl von Signalpegeln größer als der jeweilige einzelne Signalpegel ist, der Maximalwert den jeweiligen einzelnen Signalpegel ersetzt und ansonsten der jeweilige einzelne Signalpegel unverändert bleibt.

10. Verfahren nach einem der vorhergehenden Ansprüche, wobei die Modulation-Bandpassfilterung (MBF) in Schritt c) ferner eine temporale Modulations-Bandpassfilterung (TBF) umfasst, welcher die spektro-temporale Repräsentation (STR) unterzogen wird, wobei durch die temporale Modulations-Bandpassfilterung (TBF) die temporale Variation der in einem oder mehreren Zeitmodulationsfrequenzbändern (MB1, MB2, .., MB6) enthaltenen Signalanteile entlang der aufeinander folgenden Zeitfenster (T) extrahiert wird, so dass eines oder mehrere der ersten Modulationssignale (MSR') auch temporal bandpass-gefiltert sind.

11. Verfahren nach einem der vorhergehenden Ansprüche, wobei die spektrale Modulations-Bandpassfilterung (SBF) in Schritt c) eine Tiefpassfilterung der spektro-temporalen Repräsentation (STR) mit Tiefpassfiltern mit monoton zunehmender spektraler Breite entlang der Signalfrequenzen (SF1, SF2, ..., SFn) und eine Differenzbildung zwischen einer tiefpass-gefilterten Repräsentation und der benachbarten tiefpass-gefilterten Repräsentation mit der nächsthöheren spektralen Breite umfasst.

12. Vorrichtung zur rechnergestützten Verarbeitung von Audiosignalen, wobei die Vorrichtung dazu eingerichtet, ein Verfahren nach einem der vorhergehenden Ansprüche durchzuführen.

13. Vorrichtung nach Anspruch 12, wobei die Vorrichtung eine Hörhilfe ist.

**Claims**

1. A method for the computer-aided processing of audio signals, in which:

   a) an audio signal in the form of a time signal with amplitude values is digitally recorded as input signal (A), and the spectral time signals (S1, S2, ..., Sn) contained in the input signal (A) in several frequency bands (FB1, FB2, ..., FBn) with associated signal frequencies (SF1, SF2, ..., SFn) in the form of center frequencies of the respective frequency bands (FB1, FB2, ..., FBn) are determined, whereby a plurality of signal levels are obtained for each spectral time signal (S1, S2, ..., Sn);
   b) a spectro-temporal representation (STR) of the input signal (A) is determined from the spectral time signals (S1, S2, ..., Sn), which contains a plurality of signal levels for respective time windows (T), wherein the signal levels in a time window (T) are each assigned to a different signal frequency (SF1, SF2, ..., SFn);
   c) the spectro-temporal representation (STR) is subjected to modulation bandpass filtering (MBF) comprising spectral modulation bandpass filtering (SBF), wherein by the spectral modulation bandpass filtering (SBF), the spectral variation of the signal components contained in the respective modulation frequency band (MB1, MB2, ..., MB6) along the signal frequencies (SF1, SF2, ..., SFn) is extracted from several non-overlapping spectral modulation frequency bands (MB1, MB2, ..., MB5), to each of which a frequency value in the form of the minimum spectral modulation frequency in the corresponding modulation frequency band (MB1, MB2, .., MB5) is assigned, whereby for each modulation frequency band (MB1, MB2, ..., MB5) one or more first modulation signal representations (MSR) are obtained, each first modulation signal representation (MSR) containing a plurality of modulation signal levels for respective time windows (T), the modulation signal levels in a time window (T) each being assigned to a different signal frequency (SF1, SF2, ..., SFn);
   d) the first modulation signal representations (MSR) are converted into second modulation signal representations (MSR'), at least a part of the second modulation signal representations (MSR) being modified with respect to the corresponding first modulation signal representation (MSR), wherein for the modulation frequency band (MB5) with the lowest frequency value, the first modulation signal representation (MSR) is converted into the second modulation signal representation (MSR') by means of a compression function in such a way that for each signal frequency (SF1, SF2, ..., SFn) in a respective time window (T), a predetermined value range (WB) of the modulation signal levels of the first modulation signal representation (MSR) is mapped onto a reduced value range (WB') of the modulation signal levels of the second modulation signal representation (MSR'), and wherein at least one further second modulation signal representation (MSR') is obtained by multiplying the corresponding first modulation signal representation (MSR) by a factor greater than zero;

e) a modified spectro-temporal representation (STR') is determined by combining the second modulation signal representations (MSR') in such a way that for a respective time window (T) each signal level which is above a predetermined hearing threshold in the unmodified spectro-temporal representation (STR) is mapped in the modified spectro-temporal representation (STR') to a signal level within a target level range, the target level range depending on the signal frequency (SF1, SF2, ..., SFn) and is limited downwards by a lower target hearing threshold (ZS);

f) an output signal (A') modified with respect to the input signal (A) is generated from the modified spectro-temporal representation (STR').

2. The method according to claim 1, wherein the target level range is further limited upwards by an upper target hearing threshold (ZS').

3. The method according to claim 1 or 2, wherein the conversion of the first modulation signal representations (MSR) into second modulation signal representations (MSR') is carried out in step d) in such a way that all second modulation signal representations (MSR') whose respective modulation frequency band (MB1, MB2, ..., MB5) contain modulation frequencies of greater than or equal to 0.25/ERB remain unmodified or are amplified with respect to the corresponding first modulation signal representations (MSR).

4. The method according to one of the preceding claims, wherein the conversion of the first modulation signal representations (MSR) into second modulation signal representations (MSR') is carried out in step d) in such a way that for the modulation frequency band (MB1) with the highest frequency value, each second modulation signal representation (MSR') is identical to the first modulation signal representation (MSR) and one or more second modulation signal representations (MSR') comprising at least one second modulation signal representation (MSR') for the modulation frequency band (MB2) with the second-highest frequency value and/or each second modulation signal representation (MSR') for the modulation frequency band (MB5) with the lowest frequency value is modified with respect to the corresponding first modulation signal representation (MSR).

5. The method according to one of the preceding claims, wherein the second modulation signal representations (MSR') are combined in step e) in such a way that at least one second modulation signal representation (MSR') for the modulation frequency band (MB2) with the second-highest frequency value and all second modulation signal representations (MSR') for the modulation frequency band (MB1) with the highest frequency value are added to the second modulation signal representation(s) (MSR') for the modulation frequency band (MB5) with the lowest frequency value, whereupon the remaining second modulation signal representations (MSR') are added to the second modulation signal representation(s) (MSR') for the modulation frequency band (MB5) with the lowest frequency value step by step in the sequence to modulation frequency bands (MB1, MB2, ..., MB5) with low frequency values, wherein before adding a predetermined number of modulation signal levels of a second modulation signal representation (MSR') from the remaining second modulation signal representations (MSR') for a respective time window (T), it is checked whether an admissibility condition for the predetermined number of modulation signal levels is violated, the addition of the predetermined number for the respective time window (T) being terminated if the admissibility condition is violated, the admissibility condition being violated if the addition of the modulation signal level in the respective time window (T) for at least one signal frequency (SF1, SF2, ..., SFn) in the predetermined number leads to a signal level outside the target level range, which was still within the target level range before the addition.

6. The method according to one of the preceding claims, wherein the predetermined value range (WB) is limited downwards by a lower hearing threshold, which is preferably the hearing threshold (NH) in quiet of a person with normal hearing or a hearing threshold (LH) lying above it, and the reduced value range (WB') is limited downwards by a lower hearing threshold, which is preferably the hearing threshold (ZS) in quiet of a person with impaired hearing, wherein the predetermined value range (WB) and the reduced value range (WB') are preferably also limited upwards by an upper hearing threshold, wherein the upper hearing threshold for the predetermined value range (WB) is preferably a discomfort threshold (US) of a person with normal hearing and the upper hearing threshold for the reduced value range (WB) is preferably a discomfort threshold (ZS') of a person with impaired hearing.

7. The method according to one of the preceding claims, wherein in step b) the signal levels of a respective spectral time signal (S1, S2, ..., Sn) are changed in such a way that the signal levels within a respective time window (T) are replaced by a value between and including the maximum value and including the minimum value of the signal levels within the respective time window (T), preferably by the maximum value, whereby a single signal level of a respective spectral time signal (S1, S2, ..., Sn) is assigned to a time window (T).

8.  The method according to claim 7, wherein in step b) the individual signal levels of the respective spectral time signals (S 1, S2, ..., Sn) are adapted for the respective time windows (T) in such a way that the original signal level of a respective spectral time signal (S1, S2, ..., Sn) is kept constant over a predetermined number of several time windows (T) and updated original signal levels are thereby generated as long as the original signal level does not increase in the predetermined number of several time windows (T), wherein in the event that the original signal level for a time window (T) increases in the predetermined number of several time windows (T), the increased signal level for the time window (T) is used as the updated original signal level, and wherein in the event that the original signal level does not increase in the predetermined number of several time windows (T), the original signal level is successively decreased in the time windows (T) following the predetermined number of several time windows (T), thereby generating updated original signal levels until the next original signal level for a time window (T) becomes higher than the decreased signal level, whereupon the higher signal level for the time window (T) is used as the updated original signal level.

9.  The method according to claim 7 or 8, wherein in step b) in a respective time window (T), for each individual signal level of the spectral time signals (S1, S2, ..., Sn) in a copy of all signal levels of the respective time window (T), a predetermined number of signal levels is attenuated, the predetermined number of signal levels being spectrally adjacent according to the order of the signal frequencies (SF1, SF2, ..., SFn) to the respective individual signal level, wherein the attenuation is higher the greater the spectral distance of a signal level from the predetermined number of signal levels to the respective individual signal level according to the order of the signal frequencies (SF1, SF2, ..., SFn), wherein in the event that the maximum value of the attenuated signal levels of the predetermined number of signal levels is greater than the respective individual signal level, the maximum value replaces the respective individual signal level and otherwise the respective individual signal level remains unchanged.

10. The method according to one of the preceding claims, wherein the modulation bandpass filtering (MBF) in step c) further comprises a temporal modulation bandpass filtering (TBF) to which the spectro-temporal representation (STR) is subjected, wherein the temporal modulation bandpass filtering (TBF) extracts the temporal variation of the signal levels contained in one or more time modulation frequency bands (MB1, MB2, ..., MB6) along the successive time windows (T), so that one or more of the first modulation signals (MSR') are also temporally bandpass filtered.

11. The method according to one of the preceding claims, wherein the spectral modulation bandpass filtering (SBF) in step c) comprises a low-pass filtering of the spectro-temporal representation (STR) with low-pass filters with monotonically increasing spectral width along the signal frequencies (SF1, SF2, ..., SFn) and a difference formation between a low-pass filtered representation and the adjacent low-pass filtered representation with the next higher spectral width.

12. An apparatus for computer-aided processing of audio signals, the apparatus being adapted to perform a method according to any one of the preceding claims.

13. The apparatus according to claim 12, wherein the apparatus is a hearing aid.

**Revendications**

1.  Procédé de traitement assisté par ordinateur de signaux audio, lors duquel :

    a) un signal audio est détecté numériquement en tant que signal d'entrée (A) sous la forme d'un signal temporel à partir de valeurs d'amplitude et les signaux temporels spectraux (S1, S2, ..., Sn) contenus dans le signal d'entrée (A) dans plusieurs bandes de fréquences (FB1, FB2, ..., FBn) avec des fréquences de signal associées (SF1, SF2, ..., SFn) sont déterminés sous la forme de fréquences centrales des bandes de fréquences respectives (FB1, FB2, ..., FBn), moyennant quoi une pluralité de niveaux de signal pour chaque signal temporel spectral (S1, S2, ..., Sn) sont obtenus ;
    b) une représentation spectro-temporelle (STR) du signal d'entrée (A) est déterminée à partir des signaux temporels spectraux (S1, S2, ..., Sn), laquelle contient une pluralité de niveaux de signal pour des fenêtres temporelles respectives (T), dans lequel les niveaux de signal dans une fenêtre temporelle (T) sont chacun associés à une autre fréquence de signal (SF1, SF2, ..., SFn) ;
    c) la représentation spectro-temporelle (STR) est soumise à une filtration passe-bande à modulation (MBF) comprenant une filtration passe-bande à modulation spectrale (SBF), dans lequel la variation spectrale des portions de signal contenues dans la bande de fréquences de modulation respective (MB1, MB2, ..., MB6) le long des fréquences de signal (SF1, SF2, ..., SFn) est à chaque fois extraite par la filtration passe-bande à modulation spectrale (SBF) de plusieurs bandes de fréquences de modulation spectrale (MB1, MB2, ..., MB5) non chevauchantes auxquelles une valeur de fréquence est à chaque fois associée sous la forme de la fréquence de modulation spectrale minimale dans la bande de fréquences de modulation correspondante (MB1, MB2, ..., MB5), moyennant quoi une ou plusieurs premières représentations de signal de modulation (MSR) sont obtenues pour chaque bande de fréquences de modulation (MB1, MB2, ..., MB5), dans lequel chaque première représentation de signal de modulation (MSR) pour des fenêtres temporelles respectives (T) contient une pluralité de niveaux de signal de modulation, dans lequel les niveaux de signal de modulation dans une fenêtre temporelle (T) sont chacun associés à une autre fréquence de signal (SF1, SF2, ..., SFn) ;

d) les premières représentations de signal de modulation (MSR) sont transformées en secondes représentations de signal de modulation (MSR'), dans lequel au moins une partie des secondes représentations de signal de modulation (MSR') est modifiée par rapport à la première représentation de signal de modulation correspondante (MSR), dans lequel la première représentation de signal de modulation (MSR) est transformée au moyen d'une fonction de compression en la seconde représentation de signal de modulation (MSR') pour la bande de fréquences de modulation (MB5) avec la valeur de fréquence la plus basse de telle sorte que pour chaque fréquence de signal (SF1, SF2, ..., SFn) dans une fenêtre temporelle respective (T), une région de valeurs prédéfinie (WB) des niveaux de signal de modulation de la première représentation de signal de modulation (MSR) est mappée sur une région de valeurs réduite (WB') des niveaux de signal de modulation de la seconde représentation de signal de modulation (MSR'), et dans lequel au moins une autre seconde représentation de signal de modulation (MSR') est obtenue par multiplication de la première représentation de signal de modulation correspondante (MSR) avec un facteur supérieur à zéro ;

e) une représentation spectro-temporelle modifiée (STR') est déterminée en ce que les secondes représentations de signal de modulation (MSR') sont combinées de telle sorte que pour une fenêtre temporelle respective (T), chaque niveau de signal, qui se situe dans la représentation spectro-temporelle non modifiée (STR) au-dessus d'un seuil d'audition prédéfini, est mappé dans la représentation spectro-temporelle modifiée (STR') sur un niveau de signal au sein d'une plage de niveaux cibles, dans lequel la plage de niveaux cibles dépend de la fréquence de signal (SF1, SF2, ..., SFn) et est limitée vers le bas par un seuil d'audition cible inférieur (ZS) ;

f) un signal de départ (A') est généré à partir de la représentation spectro-temporelle modifiée (STR'), lequel est modifié par rapport au signal d'entrée (A).

2. Procédé selon la revendication 1, dans lequel la plage de niveaux cibles est en outre limitée vers le haut par un seuil d'audition cible supérieur (ZS').

3. Procédé selon la revendication 1 ou 2, dans lequel la transformation des premières représentations de signal de modulation (MSR) en secondes représentations de signal de modulation (MSR') à l'étape d) est réalisée de telle sorte que toutes les secondes représentations de signal de modulation (MSR') dont la bande de fréquences de modulation respective (MB1, MB2, ..., MB5) contient des fréquences de modulation supérieures ou égales à 0,25/ERB restent non modifiées ou sont amplifiées par rapport aux premières représentations de signal de modulation correspondantes (MSR).

4. Procédé selon une des revendications précédentes, dans lequel la transformation des premières représentations de signal de modulation (MSR) en secondes représentations de signal de modulation (MSR') à l'étape d) est réalisée de telle sorte que pour la bande de fréquences de modulation (MB1) avec la valeur de fréquence la plus haute, chaque seconde représentation de signal de modulation (MSR') est identique à la première représentation de signal de modulation (MSR) et une ou plusieurs secondes représentations de signal de modulation (MSR') comprenant au moins une seconde représentation de signal de modulation (MSR') est modifiée pour la bande de fréquences de modulation (MB2) avec la seconde valeur de fréquence la plus haute et/ou chaque seconde représentation de signal de modulation (MSR') est modifiée pour la bande de fréquences de modulation (MB5) avec la valeur de fréquence la plus basse par rapport à la première représentation de signal de modulation correspondante (MSR).

5. Procédé selon une des revendications précédentes, dans lequel les secondes représentations de signal de modulation (MSR') sont combinées à l'étape e) de telle sorte qu'au moins une seconde représentation de signal de modulation (MSR') pour la bande de fréquences de modulation (MB2) avec la seconde valeur de fréquence la plus haute et toutes les secondes représentations de signal de modulation (MSR') pour la bande de fréquences de modulation (MB1) avec la valeur de fréquence la plus haute sont additionnées à la ou aux secondes représentations de signal de modulation (MSR') pour la bande de fréquences de modulation (MB5) avec la valeur de fréquence la plus basse, et les secondes représentations de signal de modulation restantes (MSR') sont ensuite additionnées progressivement dans l'ordre à des bandes de fréquences de modulation (MB1, MB2, ..., MB5) avec des valeurs de fréquence basses, dans lequel avant l'addition, un nombre prédéfini de niveaux de signal de modulation d'une seconde représentation de signal de modulation (MSR') parmi les secondes représentations de signal de modulation restantes (MSR') pour une fenêtre temporelle respective (T) est contrôlé quant au fait qu'une condition d'autorisation pour le nombre prédéfini de niveaux de signal de modulation soit enfreinte, dans lequel l'addition du nombre prédéfini pour la fenêtre temporelle respective (T) est terminée en cas d'infraction de la condition d'autorisation, dans lequel la condition d'autorisation est enfreinte lorsque l'addition du niveau de signal de modulation dans la fenêtre temporelle respective (T) pour au moins une fréquence de signal (SF1, SF2, ..., SFn) dans le nombre prédéfini conduit à un niveau de signal en dehors de la plage de niveaux cibles qui se situait encore au sein de la plage de niveaux cibles avant l'addition.

6. Procédé selon une des revendications précédentes, dans lequel la région de valeurs prédéfinie (WB) est limitée vers le bas par un seuil d'audition inférieur qui est de préférence le seuil d'audition au calme (NH) d'un entendant normal ou un seuil d'audition sus-jacent (LH), et la région de valeurs réduite (WB') est limitée vers le bas par un seuil d'audition inférieur qui est de préférence le seuil d'audition au calme (ZS) d'un malentendant, dans lequel la région de valeurs prédéfinie (WB) et la région de valeurs réduite (WB') sont de préférence également limitées vers le haut par un seuil d'audition supérieur, dans lequel le seuil d'audition supérieur pour la région de valeurs prédéfinie (WB) est de préférence un seuil d'inconfort (US) d'un entendant normal et le seuil d'audition supérieur pour la région de valeurs réduite (WB') est de préférence un seuil d'inconfort (ZS') d'un malentendant.

7. Procédé selon une des revendications précédentes, dans lequel les niveaux de signal d'un signal temporel spectral respectif (S1, S2, ..., Sn) à l'étape b) sont modifiés de telle sorte que les niveaux de signal sont remplacés au sein d'une fenêtre temporelle respective (T) par une valeur entre la valeur maximale incluse et la valeur minimale incluse des niveaux de signal au sein de la fenêtre temporelle respective (T), de préférence par la valeur maximale, moyennant quoi un niveau de signal individuel d'un signal temporel spectral respectif (S1, S2, ..., Sn) est associé à une fenêtre temporelle (T).

8. Procédé selon la revendication 7, dans lequel les niveaux de signal individuels des signaux temporels spectraux respectifs (S1, S2, ..., Sn) pour les fenêtres temporelles respectives (T) sont adaptés à l'étape b) de telle sorte que le niveau de signal d'origine d'un signal temporel spectral respectif (S1, S2, ..., Sn) est maintenu constant par le biais d'un nombre prédéterminé de plusieurs fenêtres temporelles (T) et des niveaux de signal d'origine actualisés sont de ce fait générés tant que le niveau de signal d'origine n'augmente pas dans le nombre prédéterminé de plusieurs fenêtres temporelles (T), dans lequel au cas où le niveau de signal d'origine pour une fenêtre temporelle (T) augmente dans le nombre prédéterminé de plusieurs fenêtres temporelles (T), le niveau de signal accru pour la fenêtre temporelle (T) est réutilisé en tant que niveau de signal d'origine actualisé, et dans lequel au cas où le niveau de signal d'origine n'augmente pas dans le nombre prédéterminé de plusieurs fenêtres temporelles (T), le niveau de signal d'origine est successivement abaissé dans les fenêtres temporelles (T) qui suivent le nombre prédéterminé de plusieurs fenêtres temporelles (T) et des niveaux de signal d'origine actualisés sont de ce fait générés jusqu'à ce que le niveau de signal d'origine suivant soit supérieur pour une fenêtre temporelle (T) au niveau de signal abaissé, après quoi le niveau de signal supérieur pour la fenêtre temporelle (T) est réutilisé en tant que niveau de signal d'origine actualisé.

9. Procédé selon la revendication 7 ou 8, dans lequel un nombre prédéterminé de niveaux de signal qui sont voisins spectralement conformément à l'ordre des fréquences de signal (SF1, SF2, ..., SFn) du niveau de signal individuel respectif est amorti à l'étape b) dans une fenêtre temporelle respective (T) pour chaque niveau de signal individuel des signaux temporels spectraux (S1, S2, ..., Sn) dans une copie de tous les niveaux de signal de la fenêtre temporelle respective (T), dans lequel l'amortissement est d'autant plus élevé que l'écart spectral d'un niveau de signal parmi le nombre prédéterminé de niveaux de signal est grand par rapport au niveau de signal individuel respectif conformément à l'ordre des fréquences de signal (SF1, SF2, ..., SFn), dans lequel au cas où la valeur maximale des niveaux de signal amortis du nombre prédéterminé de niveaux de signal est supérieure au niveau de signal individuel respectif, la valeur maximale remplace le niveau de signal individuel respectif et sinon le niveau de signal individuel respectif reste inchangé.

10. Procédé selon une des revendications précédentes, dans lequel la filtration passe-bande à modulation (MBF) à l'étape c) comprend en outre une filtration passe-bande à modulation temporelle (TBF) à laquelle la représentation spectro-temporelle (STR) est soumise, dans lequel la variation temporelle des portions de signal contenues dans une ou plusieurs bandes de fréquences de modulation temporelle (MB1, MB2, ..., MB6) le long des fenêtres temporelles successives (T) est extraite par la filtration passe-bande à modulation temporelle (TBF) de sorte qu'un ou plusieurs des premiers signaux de modulation (MSR') subissent également une filtration passe-bande temporelle.

11. Procédé selon une des revendications précédentes, dans lequel la filtration passe-bande à modulation spectrale (SBF) à l'étape c) comprend une filtration passe-bas de la représentation spectro-temporelle (STR) avec des filtres passe-bas avec une largeur spectrale à croissance monotone le long des fréquences de signal (SF1, SF2, ..., SFn) et une formation de différence entre une représentation à filtration passe-bas et la représentation à filtration passe-bas voisine avec la largeur spectrale supérieure suivante.

12. Dispositif de traitement assisté par ordinateur de signaux audio, dans lequel le dispositif est configuré pour réaliser un procédé selon une des revendications précédentes.

13. Dispositif selon la revendication 12, dans lequel le dispositif est une aide auditive.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006047694 B4
- DE 102004044565 A1
- WO 2015113601 A1
- EP 1912470 A2
- US 20110150229 A1

**In der Beschreibung aufgeführte Nicht-Patentliteratur**

- **SCHÄDLER MARC RENÉ et al.** A simulation framework for auditory discrimination experiments: Revealing the importance of across-frequency processing in speech perception. *THE JOURNAL OF THE ACOUSTICAL SOCIETY OF AMERICA,* 13. Mai 2016, vol. 139 (5), 2708-2722